# EUROPEAN PATENT SPECIFICATION

(11) **EP 2 861 087 B1**
(45) Date of publication and mention of the grant of the patent: **24.07.2019**
(21) Application number: 13737679.4
(22) Date of filing: 18.06.2013
(51) Int. Cl.: A61K 31/201, A61K 31/683, A61K 35/20, A23L 33/10, A23L 33/115, A23L 33/19, A23L 33/21, A23L 33/00, A23L 33/12

(54) **METABOLIC IMPRINTING EFFECTS OF NUTRITION WITH LARGE LIPID GLOBULES COMPRISING MILK FAT AND VEGETABLE FAT**
AUSWIRKUNG AUF METABOLISCHE PRÄGUNG DER ERNÄHRUNG GROSSEN LIPIDKÜGELCHEN AUS MILCHFETT UND PFLANZLICHEM FETT
EFFETS D'EMPREINTE MÉTABOLIQUE D'UNE NUTRITION INCLUANT DES GLOBULES GRAS DE GRANDE TAILLE COMPRENANT DES MATIÈRES GRASSES DU LAIT ET DES MATIÈRES GRASSES VÉGÉTALES

(30) Priority: 18.06.2012 WO PCT/NL2012/050426
(43) Date of publication of application: 22.04.2015
(73) Proprietor: N.V. Nutricia, 2712 HM Zoetermeer (NL)
(72) Inventor: VAN DER BEEK, Eline Marleen, NL-3584 CT Utrecht (NL); ABRAHAMSE-BERKEVELD, Marieke, NL-3584 CT Utrecht (NL); OOSTING, Annemarie, NL-2403 HL Alphen aan den Rijn (NL); ACTON, Dennis Stanley, NL-3554 ED Utrecht (NL); MENSINK, Johannes Lambertus Maria, NL-2611 JP Delft (NL)
(74) Representative: Nederlandsch Octrooibureau
(86) International application number: PCT/NL2013/050431
(87) International publication number: WO 2013/191542

(56) References cited:
- WO-A1-2010/027258
- WO-A1-2010/068086

## Description

### FIELD OF THE INVENTION

The present invention relates to the field of infant nutrition and the effect of the lipid component of the early in life diet on the health and body composition later in life.

### BACKGROUND OF THE INVENTION

Breast-feeding is the preferred method of feeding infants. Breast fed infants have a decreased chance of becoming obese later in life, compared to standard formula fed infants, but little is known about the effects of ingredients in the infant formulae on obesity later in life. Obesity is a major health problem in the Western world. It is a medical condition in which excess fat has accumulated to the extent that it may have an adverse effect on health, leading to reduced life expectancy and it is associated with many diseases, particularly heart disease and type 2 diabetes. Obesity is a leading preventable cause of death worldwide, with increasing prevalence in adults and children, and authorities view it as one of the most serious public health problems of the 21^{st} century.

Infant formulae comprise predominantly vegetable oils as lipid source, since the fatty acid composition of vegetable oils has a lower amount of saturated fatty acids and a higher amount of polyunsaturated fatty acid than present in cow's milk fat. This fatty acid composition is considered more healthy and more close to human milk.

Infant nutrition with lipid with a fatty acid composition preventing obesity later in life are known. WO 2007/073192 relates to infant formulae comprising a low amount of linoleic acid and a low weight ratio of linoleic acid to alpha-linolenic acid and LC-PUFA for the prevention of obesity later in life. WO 2007/073193 relates to infant formulae with specific linoleic acid to alpha-linolenic acid ratio's, low linoleic acid content and comprising phospholipids, sphingolipids, cholesterol and/or choline plus uridine for the prevention of obesity later in life.

The lipid component in infant formulae typically is present in the form of small lipid globules, which are obtained after a severe homogenization step. Small lipid globules are desired because it results in a stable, non creaming, emulsion of lipid globules. The globules have a large surface area and because of the low amount of polar lipids, such as phospholipids, in the vegetable oils, the surface of the lipid globules is coated mainly with proteins, typically casein.

WO 2010/0027258 and WO 2010/0027259 relate to infant formulae with large lipid globules coated with phospholipids for the prevention of obesity later in life. WO 2010/068105 discloses nutritional compositions for infants with a large lipid globule size.

WO 2005/051091 discloses a lipid preparation, to be included in infant formulae as a beneficial ingredient *per se* and for improving cognitive and vision development in particular, that is organised in a globular microstructure naturally occurring in human milk.

WO2009/138680 discloses compositions for infants with a dairy fat and a vegetable fat source.

The present invention aims to provide infant nutrition with a lipid component beneficially effecting health and/or the body composition later in life.

### SUMMARY OF THE INVENTION

It was found that administration early in life of a diet comprising a lipid component in the form of large lipid globules comprising a mixture of milk fat and vegetable fat beneficially affects the growth and body composition later in life. When early in life a diet of the present invention was consumed that comprised large lipid globules of a mixture of milk fat and vegetable oil, it was observed that later in life the body composition was changed, resulting in less fat mass accumulation, less fat mass relative to total body weight, increased lean body mass, increased bone mass and bone mineral density when compared to the body composition upon administering early in life a control diet with small lipid globules comprising a mixture of milk and vegetable fat, or a control diet with large lipid globules with vegetable fat. This is surprising, since the overall fatty acid composition in the diets was very similar except for the amount of short chain fatty acids. This is surprising, since the later in life diet was for all groups the same Western style diet.

So due to the presence in the diet of a mixture of vegetable and milk fat in the form of large lipid globules consumed during infancy differences in patterns of growth arise and the body is programmed differently which results in a healthier body composition later in life, i.e. adulthood. The body is programmed to resist a relatively obesogenic Western style diet.

The present invention hence relates to nutritional compositions, in particular formulae for infants or growing up milks for toddlers, comprising a lipid component with large lipid globules, and a mixture of milk fat and vegetable fat. In order to reconstitute the lipid globules during the manufacture of the nutritional composition the milk fat source is preferably butter, butteroil or anhydrous milk fat.

The present invention therefore can be advantageously used for food compositions intended for infants and/or toddlers and preferably is intended to be consumed early in life in order to prevent obesity, visceral obesity, decrease or prevent insulin resistance, prevent diabetes type 2, prevent metabolic syndrome, prevent cardio- or cerebrovascular diseases, increase lean body mass, increase muscle tissue, decrease relative fat mass, decrease fat mass accumulation, increased bone mass and/or increased bone mineral density later in life.

### DETAILED DESCRIPTION OF THE INVENTION

The invention concerns a nutritional composition comprising carbohydrates, protein and lipid, wherein the lipid is present in the form of lipid globules, the lipid globules have a volume mode diameter of 1.0 µm or above, wherein the lipid has a fatty acid composition comprising:
i) 0.9 to 4 wt.% short chain fatty acids (SCFA) being the sum of butyric acid (BA) and caproic acid (CA) based on total fatty acids, and wherein the composition comprises between 0.75 and 4 wt.% butyric acid based on weight of the total fatty acyl chains,
ii) 10 to 25 wt.% poly-unsaturated fatty acids (PUFA), based on total fatty acids, comprising linoleic acid (LA) and alpha-linolenic acid (ALA) in a weight ratio of 2 to 10, wherein the lipid is one or more selected from the group consisting of triglycerides, polar lipids, free fatty acids, mono- and diglycerides, and wherein the lipid comprises 0.5 - 20 wt.% phospholipids based on total lipid.

The invention also concerns a nutritional composition as defined herein, for use in one or more selected from prevention of obesity, reducing the risk of obesity, treatment of obesity, prevention of diabetes type 2, reducing the risk of occurrence of insulin resistance and improving insulin sensitivity, preventing metabolic syndrome and preventing of osteopenia and/or osteoporosis and/or reducing the risk of occurrence of osteopenia and/or osteoporosis.

The invention also concerns a method for improving body composition, preferably a non-therapeutic method for improving body composition, the improvement of body composition being selected from the group consisting of increased lean body mass, decreased fat mass relative to total body weight, decreased visceral fat mass relative to total body weight, decreased visceral fat mass relative to total fat mass, decreased fat accumulation, increased muscle mass, increased bone mass, and increased bone mineral density, comprising administering a nutritional composition as defined herein.

Obesity is considered to be a medical condition and thus prevention of obesity, reducing the risk of obesity, and/or treatment of obesity is seen as a method of treatment of the human body by therapy. This includes visceral obesity. Human subjects may suffer from visceral obesity, but not from overall obesity. This situation is described as thin on the outside, fat on the inside, which is abbreviated in the art as TOFI. A person suffering from visceral obesity is more at risk of becoming diabetic, even though overall obesity is not present. Thus visceral obesity is considered to be a medical condition as well and thus prevention of visceral obesity, reducing the risk of visceral obesity, and/or treatment of visceral obesity is seen as a method of treatment of the human body by therapy. However, improving body composition can be considered as not being therapeutic meaning that for all jurisdictions this aspect can be properly worded by the method, more in particular a non-therapeutic method, for improving body composition as specified above.

Diabetes mellitus type 2, also referred to as diabetes type 2, is considered to be a medical condition and thus prevention of diabetes type 2, reducing the risk of diabetes type 2, and/or treatment of diabetes type 2 is seen as a method of the human body by therapy. Likewise, decreasing insulin resistance, or the preventing occurrence of insulin resistance, or improving insulin sensitivity, is seen as a medical treatment.

### Lipid component

The composition according to the present invention or that is to be administered according to the present method or use comprises lipids. Lipids in the present invention are one or more selected from the group consisting of triglycerides, polar lipids (such as phospholipids, cholesterol, glycolipids, sphingomyelin), free fatty acids, mono- and diglycerides. Preferably the composition comprises at least 70 wt. %, more preferably at least 80 wt.%, more preferably at least 85 wt.% triglycerides, even more preferably at least 90 wt% triglycerides based on total lipids.

The lipid provides preferably 30 to 60 % of the total calories of the composition. More preferably the present composition comprises lipid providing 35 to 55 % of the total calories, even more preferably the present composition comprises lipid providing 40 to 50 % of the total calories. When in liquid form, e.g. as a ready-to-feed liquid, the composition preferably comprises 2.1 to 6.5 g lipid per 100 ml, more preferably 3.0 to 4.0 g per 100 ml. Based on dry weight the present composition preferably comprises 10 to 50 wt.% lipid, more preferably 12.5 to 40 wt.% lipid, even more preferably 19 to 30 wt.% lipid. The lipid comprises preferably from 80 to 100 wt.% triglycerides based on total lipid, more preferably 90 to 100 wt.%.

The present composition also may comprise non-vegetable lipids and non-milk fat, such as animal fat other than milk fat, such as fish oil, and egg lipid, and microbial, algal, fungal or single cell oils. Preferably the non vegetable, non-milk fat is present in an amount of at most 10 wt.% based on total lipid, more preferably at most 5 wt.%. Preferably the lipid in the nutritional composition according to the invention comprises a fat source comprising long chain poly-unsaturated fatty acids (LC-PUFA), selected from the group consisting of fish oil, marine oil, algal oil, microbial oil, single cell oil and egg lipid in an amount of 0.25 to 10 wt% based on total lipid, preferably in an amount of 0.5 to 10 wt%.

### Fatty acid composition

Herein LA refers to linoleic acid and/or acyl chain (18:2 n6); ALA refers to α-linolenic acid and/or acyl chain (18:3 n3); LC-PUFA refers to long chain polyunsaturated fatty acids and/or acyl chains comprising at least 20 carbon atoms in the fatty acyl chain and with 2 or more unsaturated bonds; DHA refers to docosahexaenoic acid and/or acyl chain (22:6, n3); EPA refers to eicosapentaenoic acid and/or acyl chain (20:5 n3); ARA refers to arachidonic acid and/or acyl chain (20:4 n6); DPA refers to docosapentaenoic acid and/or acyl chain (22:5 n3). PUFA refers to polyunsaturated fatty acids and/or acyl chains comprising 2 or more unsaturated bonds; MUFA refers to fatty acids and/or acyl chains comprising one unsaturated bond; SFA refers to saturated fatty acids and/or acyl chains. Medium chain fatty acids (MCFA) refer to fatty acids and/or acyl chains with a chain length of 8 to 12 carbon atoms. Short chain fatty acids (SCFA) refers to fatty acids and/or acyl chains with 4 or 6 carbon atoms. Butyric acid (BA) refer to fatty acids and/or acyl chains comprising 4 carbon atoms, caproic (CA) refer to fatty acids and/or acyl chains comprising 6 carbon atoms.

The present composition comprises 10 to 25 wt.% PUFA based on total fatty acids. The presence of such an amount of PUFA above 10 wt % is thought to be essential in order to have the health effects on body composition, obesity, diabetes, metabolic syndrome and osteoporosis, as claimed in the present invention. Amounts above 25 wt% will be much higher than present in human milk, and will encompass technological problems such as stability in the nutritional composition.

LA preferably is present in a sufficient amount in order to promote a healthy growth and development, yet in an amount as low as possible to prevent occurrence of obesity later in life. The composition therefore preferably comprises less than 15 wt.% LA based on total fatty acids, preferably between 5 and 14.5 wt.%, more preferably between 6 and 10 wt.%. Preferably the composition comprises over 5 wt.% LA based on fatty acids. Preferably ALA is present in a sufficient amount to promote a healthy growth and development of the infant. The present composition therefore preferably comprises at least 1.0 wt.% ALA based on total fatty acids. Preferably the composition comprises at least 1.5 wt.% ALA based on total fatty acids, more preferably at least 2.0 wt.%. Preferably the composition comprises less than 10 wt.% ALA, more preferably less than 5.0 wt.% based on total fatty acids. The weight ratio LA/ALA should be well balanced in order to prevent obesity, while at the same time ensuring a normal growth and development. Therefore, the present composition comprises a weight ratio of LA/ALA from 2 to 10, more preferably from 3 to 7, more preferably from 3 to 6, even more preferably from 4 to 5.5, even more preferably from 4 to 5.

In one embodiment the present nutritional composition comprises between 0.6 to 4.0 wt% SCFA based on total fatty acids, wherein SCFA is the sum of BA and CA. The presence of relatively high amounts of CA and BA is characteristic for triglycerides derived from ruminant milk, such as cow's milk. It is not present in vegetable fat or MCT rich fat such as coconut oil and it is also not found in polar lipids derived from milk fat. So one alternative way of describing the presence of milk fat triglycerides in a composition is to define the fatty acid profile in having a SCFA content of between 0.6 and 4.0 wt.% based total fatty acids. Without wishing to be bound by theory, the presence of such SCFA may provide the advantageous effects. Thus, according to the present invention, the lipid comprised in the nutritional composition comprises between 0.9 to 4.0 wt% of the sum of BA and CA and 10 to 25 wt.% PUFA based on total fatty acids comprising LA and ALA in a weight ratio of 2 to 10.

Based on total weight of fatty acyl chains, the present nutritional composition preferably comprises at least 1.2 wt. % short chain fatty acyl chains being the sum of BA and CA, more preferably at least 2.0 wt.%. The present nutritional composition has a wt.% of short chain fatty acyl chains below 4 wt.% based on weight of total fatty acyl chains more preferable below 3 wt.%.

The present composition contains between 0.75 and 4 wt.% butyric acid based on based on weight of total fatty acyl chains, preferably between 0.75 and 2.5 wt.%, even more preferably between 0.75 and 2 wt.%.

Since MCFA contribute to a reduced fat mass later in life when administered to an infant, the present composition preferably comprises at least 5 wt.% MCFA based on total fatty acids, more preferably at least 7 wt%. Since MCFA reduces body fat deposition with no preference for central fat mass, and since MFCA does not decrease the number of adipocytes, the present composition advantageously comprises less than 15 wt.% MCFA based on total fatty acids, more preferably less than 10 wt.%.

Preferably the present composition comprises LC-PUFA, more preferably n-3 LC-PUFA, since n-3 LC-PUFA reduce obesity later in life, more preferably central obesity. More preferably, the present composition comprises EPA, DPA and/or DHA, even more preferably DHA. Since a low concentration of DHA, DPA and/or EPA is already effective and normal growth and development are important, the content of n-3 LC-PUFA in the present composition, more preferably DHA, preferably does not exceed 5 wt.% of the total fatty acid content. Preferably the present nutritional composition comprises at least 0.15 wt.%, preferably at least 0.35 wt.%, more preferably at least 0.75 wt.%, n-3 LC-PUFA, more preferably DHA, of the total fatty acid content. The present nutritional composition preferably comprises at least 0.25 wt% LC-PUFA based on total fatty acids. Preferably the lipid in the nutritional composition according to the invention comprises a fat source comprising 0.25 wt. % to 5 wt.% LC-PUFA based on total fatty acids of which at least 0.15 wt% n-3 LC-PUFA based on total fatty acids selected from the group consisting of DHA, EPA, and DPA, more preferably DHA.

As the group of n-6 fatty acids, especially arachidonic acid (ARA) and LA as its precursor, counteracts the group of n-3 fatty acids, especially DHA and EPA and ALA as their precursor, the present composition comprises relatively low amounts of ARA. The n-6 LC-PUFA, more preferably ARA, content preferably does not exceed 5 wt.%, more preferably does not exceed 2.0 wt.%, more preferably does not exceed 0.75 wt.%, even more preferably does not exceed 0.5 wt.%, based on total fatty acids. Since ARA is important in infants for optimal functional membranes, especially membranes of neurological tissues, the amount of n-6 LC-PUFA, preferably of ARA, is preferably at least 0.02 wt.% more preferably at least 0.05 wt.%, more preferably at least 0.1 wt.% based on total fatty acids, more preferably at least 0.2 wt.%. The presence of ARA is advantageous in a composition low in LA since it remedies LA deficiency. The presence of preferably low amounts of ARA is beneficial in nutrition to be administered to infants below the age of 6 months, since for these infants the infant formulae is generally the only source of nutrition. Preferably the n-6 LC-PUFA/n-3 LC-PUFA weight ratio, more preferably ARA/DHA weigh ratio is below 3, more preferably 2 or below, even more preferably 1 or below. A low n-6 LC-PUFA/n-3 LC-PUFA, more preferably ARA/DHA, ratio is especially preferred when LA/ALA ratio's are above 5, more preferably above 7, in order to counterbalance.

### Lipid globule size

According to the present invention, lipid is present in the composition in the form of lipid globules. When in liquid form these lipid globules are emulsified in the aqueous phase. Alternatively the lipid globules are present in a powder and the powder is suitable for reconstitution with water or another food grade aqueous phase. The lipid globules comprise a core and a surface. The core preferably comprises vegetable fat and milk fat and preferably comprises at least 90 wt.% triglycerides and more preferably essentially consists of triglycerides. Not all triglyceride lipids that are present in the composition need necessarily be comprised in the core of lipid globules, but preferably a major part is, preferably more than 50% wt.%, more preferably more than 70 wt.%, even more preferably more than 85 wt.%, even more preferably more than 95 wt.%, most preferably more than 98 wt.% of the triglyceride lipids that are present in the composition are comprised in the core of lipid globules.

The lipid globules of the present invention have a volume-weighted mode diameter above 1.0 µm, preferably above 2.0 µm, more preferably above 3.0 µm, more preferably 4.0 µm or above, preferably between 1.0 and 10 µm, more preferably between 2.0 and 8.0 µm, even more preferably between 3.0 and 8.0 µm, most preferably between 4.0 µm and 8.0 µm. Preferably in addition the size distribution is in such a way that at least 45 volume %, preferably at least 55 volume %, even more preferably at least 65 volume %, even more preferably at least 75 volume % has a diameter between 2 and 12 µm. More preferably at least 45 volume %, preferably at least 55 volume %, even more preferably at least 65 volume %, even more preferably at least 75 volume % has a diameter between 2 and 10 µm. Even more preferably at least 45 volume %, preferably at least 55 volume %, even more preferably at least 65 volume %, even more preferably at least 75 volume % has a diameter between 4 and 10 µm. Preferably less than 5 volume % has a diameter above 12 µm.

Standard infant formulae or growing up milks have lipid globules with mode diameter below 0.5 µm. It was found that large lipid globules have an improved effect on obesity later in life.

The percentage of lipid globules is based on volume of total lipid. The mode diameter relates to the diameter which is the most present based on volume of total lipid, or the peak value in a graphic representation, having on the X-as the diameter and on the Y-as the volume (%).

The volume of the lipid globule and its size distribution can suitably be determined using a particle size analyzer such as a Mastersizer (Malvern Instruments, Malvern, UK), for example by the method described in Michalski et al, 2001, Lait 81: 787-796.

### Coating with phospholipids

The present invention comprises polar lipids. Polar lipids are amphipathic of nature and include glycerophospholipids, glycosphingolipids, sphingomyelin and/or cholesterol. The composition according to the present invention comprises phospholipids (the sum of glycerophospholipids and sphingomyelin). Polar lipids in the present invention relate to the sum of glycerophospholipids, glycosphingolipids, sphingomyelin and cholesterol. Polar lipids, more preferably phospholipids, are preferably present as a coating on the surface of the lipid globule. By 'coating' is meant that the outer surface layer of the lipid globule comprises polar lipids, whereas these polar lipids are virtually absent in the core of the lipid globule. The presence of polar lipids, in particular, phospholipids as a coating or outer layer of the lipid globule in the diet administered early in life was found to advantageously further decrease fat mass, decrease relative fat mass, i.e. obesity, and/or increase lean body mass later in life. Thus in one embodiment the coating preferably comprises phospholipids and/or polar lipids. Not all phospholipids and/or polar lipids that are present in the composition need necessarily be comprised in the coating, but preferably a major part is. Preferably more than 50 wt.%, more preferably more than 70 wt,%, even more preferably more than 85 wt.%, most preferably more than 95 wt.% of the phospholipids and/or polar lipids that are present in the composition are comprised in the coating of lipid globules.

In one embodiment, in the nutritional composition according to the invention the lipid globules have a volume mode diameter of 1.0 µm or above, and are at least partly coated on the surface with phospholipids, the amount of phospholipids present in the nutritional composition being from 0.5 to 20 wt.% phospholipids based on total lipid. A combination of large lipid size and coating has a further improved effect on preventing obesity later in life.

The present composition preferably comprises glycerophospholipids. Glycerophospholipids are a class of lipids formed from fatty acids esterified at the hydroxyl groups on carbon-1 and carbon-2 of the backbone glycerol moiety and a negatively-charged phosphate group attached to carbon-3 of the glycerol via an ester bond, and optionally a choline group (in case of phosphatidylcholine, PC), a serine group (in case of phosphatidylserine, PS), an ethanolamine group (in case of phosphatidylethanolamine, PE), an inositol group (in case of phosphatidylinositol, PI) or a glycerol group (in case of phosphatidylglycerol, PG) attached to the phosphate group. Lysophospholipids are a class of phospholipids with one fatty acyl chain. Preferably the present composition contains PC, PS, PI and/or PE, more preferably at least PC. The present composition preferably comprises sphingomyelin. Sphingomyelins have a phosphorylcholine or phosphorylethanolamine molecule esterified to the 1-hydroxy group of a ceramide. Preferably the present nutritional composition comprisises 0.05 to 10 wt.% sphingomyelin based on total lipid, more preferably 0.1 to 5 wt%, even more preferably 0.2 to 2 wt.%. Sphingomyelin is classified as phospholipid as well as sphingolipid, but are not classified as a glycerophospholipid nor as a glycosphingolipid.

The present composition preferably comprises glycosphingolipids. The term glycosphingolipids as in the present invention particularly refers to glycolipids with an amino alcohol sphingosine. The sphingosine backbone is O-linked to a charged headgroup such as ethanolamine, serine or choline backbone. The backbone is also amide linked to a fatty acyl group. Glycosphingolipids are ceramides with one or more sugar residues joined in a β-glycosidic linkage at the 1-hydroxyl position. Preferably the present composition contains gangliosides, more preferably at least one ganglioside selected from the group consisting of GM3 and GD3.

Sphingolipids are in the present invention defined as the sum of sphingomyelin and glycosphingolipids. Phospholipids are in the present invention defined as the sum of sphingomyelin and glycerophospholipids. Preferably the phospholipids are derived from milk lipids. Preferably the weight ratio of phospholipids : glycosphingolipids is from 2:1 to 10:1, more preferably 2:1 to 5:1.

The present composition comprises 0.5 to 20 wt.% phospholipids based on total lipid, more preferably 0.5 to 10 wt.%, more preferably 1 to 10 wt.%, even more preferably 2 to 10 wt.% even more preferably 3 to 8 wt.% phospholipids based on total lipid. Preferably the present composition comprises 0.1 to 10 wt.% glycosphingolipids based on total lipid, more preferably 0.5 to 5 wt.%, even more preferably 2 to 4 wt%. Preferably the present composition comprises 0.5 to 10 wt.% (glycosphingolipids plus phospholipids) based on total lipid, more preferably 1.0 to 10 wt.% (glycosphingolipids plus phospholipids) based on total lipid.

The present composition preferably comprises cholesterol. The present composition preferably comprises at least 0.005 wt.% cholesterol based on total lipid, more preferably at least 0.02 wt.%, more preferably at least 0.05 wt.%., even more preferably at least 0.1 wt.%. Preferably the amount of cholesterol does not exceed 10 wt.% based on total lipid, more preferably does not exceed 5 wt.%, even more preferably does not exceed 1 wt.% of total lipid.

Preferably the present composition comprises 0.6 to 25 wt.% polar lipids based on total lipid, wherein the polar lipids are the sum of phospholipids, glycosphingolipids, and cholesterol, more preferably 0.6 to 12 wt.%, more preferably 1 to 10 wt.%, even more preferably 2 to 10 wt%, even more preferably 3.0 to 10 wt.% polar lipids based on total lipid, wherein the polar lipids are the sum of phospholipids, glycosphingolipids, and cholesterol.

Preferred sources for providing the phospholipids, glycosphingolipids and/or cholesterol are egg lipids, milk fat, buttermilk fat and butter serum fat (such as beta serum fat). A preferred source for phospholipids, particularly PC, is soy lecithin and/or sunflower lecithin. The present composition preferably comprises phospholipids derived from mammalian milk. Preferably the present composition comprises phospholipids and glycosphingolipids derived from milk. Preferably also cholesterol is obtained from milk. Preferably the polar lipids, in particular phospholipids, are derived from milk. Polar lipids, in particular phospholipids, derived from milk include the polar lipids, in particular phospholipids, isolated from milk lipid, cream lipid, cream serum lipid, butter serum lipid (beta serum lipid), whey lipid, cheese lipid and/or buttermilk lipid. The buttermilk lipid is typically obtained during the manufacture of buttermilk. The butter serum lipid or beta serum lipid is typically obtained during the manufacture of anhydrous milk fat from butter. Preferably the phospholipids, glycosphingolipids and/or cholesterol are obtained from milk cream. The composition preferably comprises phospholipids, glycosphingolipids and/or cholesterol from milk of cows, mares, sheep, goats, buffalos, horses and camels. It is most preferred to use a lipid extract isolated from cow's milk. The use of polar lipids from milk fat advantageously comprises the polar lipids from milk fat globule membranes, which are more reminiscent to the situation in human milk. Polar lipids, in particular phospholipids, derived from fat milk advantageously decrease fat mass to a larger extent than polar lipids from other sources. The polar lipids, in particular phospholipids, are located on the surface of the lipid globule, i.e. as a coating or outer layer. It was found that when the polar lipids or phospholipids are present in the coating of the lipid globule they are more effective than when they are dry blended into the powdered product, i.e. present as ingredient as such. A suitable way to determine whether the polar lipids are located on the surface of the lipid globules is laser scanning microscopy. The concomitant use of polar lipids in particular phospholipids, derived from domestic animals milk and trigycerides derived from vegetable lipids therefore enables to manufacture coated lipid globules with a coating more similar to human milk, while at the same time providing an optimal fatty acid profile. Suitable commercially available sources for milk polar lipids are BAEF, SM2, SM3 and SM4 powder of Corman, Salibra of Glanbia, and LacProdan MFGM-10 or PL20 from Arla. Preferably the source of milk polar lipids comprises at least 4 wt.% phospholipids based on total lipid, more preferably 7 to 75 wt.%, most preferably 20 to 70 wt.% phospholipids based on total lipid. Preferably the weight ratio phospholipids to protein is above 0.10, more preferably above 0.20, even more preferably above 0.3. Preferably at least 25 wt.%, more preferably at least 40 wt.%, most preferably at least 75 wt.% of the polar lipids, in particular phospholipids, is derived from milk polar lipids.

Methods for obtaining lipid globules with an increased size and/or coating with phospholipids are disclosed in WO 2010/0027258 and WO 2010/0027259.

### Process

The invention also concerns a process for the manufacture of a nutritional composition according to the invention comprising preparing an aqueous phase comprising protein and digestible carbohydrates and preparing a fat phase comprising vegetable lipid and mammalian milk lipid in a weight ratio of 3/7 to 9/1, wherein the fatty acid composition of the lipid comprises 10 to 25 wt.% poly-unsaturated fatty acids (PUFA), based on total fatty acids, comprising linoleic acid (LA) and alpha-linolenic acid (ALA) in a weight ratio of 2 to 10, comprising 0.6 to 4 wt.% short chain fatty acids (SCFA) being the sum of butyric acid (BA) and caproic acid (CA) based on total fatty acids, adding the fat and aqueous phase together and homogenizing the mixture of fat and aqueous phase into an oil-in-water emulsion with lipid globules having a volume mode diameter of 1.0 µ m or above.

Further, the invention relates to the product, preferably the nutritional composition obtained by the process according to the invention for any of the uses or methods according to the invention.

### Nutritional composition

### Digestible carbohydrates

The composition comprises digestible carbohydrate. The digestible carbohydrate preferably provides 30 to 80% of the total calories of the composition. Preferably the digestible carbohydrate provides 40 to 60% of the total calories. When in liquid form, e.g. as a ready-to-feed liquid, the composition preferably comprises 3.0 to 30 g digestible carbohydrate per 100 ml, more preferably 6.0 to 20, even more preferably 7.0 to 10.0 g per 100 ml. Based on dry weight the present composition preferably comprises 20 to 80 wt.%, more preferably 40 to 65 wt.% digestible carbohydrates.

Preferred digestible carbohydrate sources are lactose, glucose, sucrose, fructose, galactose, maltose, starch and maltodextrin. Lactose is the main digestible carbohydrate present in human milk. Lactose advantageously has a low glycemic index. The present composition preferably comprises lactose. The present composition preferably comprises digestible carbohydrate, wherein at least 35 wt.%, more preferably at least 50 wt.%, more preferably at least 75 wt.%, even more preferably at least 90 wt.%, most preferably at least 95 wt.% of the digestible carbohydrate is lactose. Based on dry weight the present composition preferably comprises at least 25 wt.% lactose, preferably at least 40 wt.%.

### Non digestible carbohydrates

In one embodiment the present composition comprises non-digestible oligosaccharides with a degree of
polymerization (DP) between 2 and 250, more preferably 3 and 60. The non-digestible oligosaccharides advantageously prevent the onset of insulin resistance.

Preferably the present composition comprises fructo-oligosaccharides, galacto-oligosaccharides and/or galacturonic acid oligosaccharides, more preferably galacto-oligosaccharides, most preferably transgalacto-oligosaccharides. In a preferred embodiment the composition comprises a mixture of transgalacto-oligosaccharides and fructo-oligosaccharides. Suitable non-digestible oligosaccharides are for example Vivinal GOS (Frieslandcampina DOMO), Raftilin HP or Raftilose (Orafti).

Preferably, the composition comprises of 80 mg to 2 g non-digestible oligosaccharides per 100 ml, more preferably 150 mg to 1.50 g, even more preferably 300 mg to 1 g per 100 ml. Based on dry weight, the composition preferably comprises 0.25 wt.% to 20 wt.%, more preferably 0.5 wt.% to 10 wt.%, even more preferably 1.5 wt.% to 7.5 wt.%. A lower amount of non-digestible oligosaccharides will be less effective in preventing obesity, whereas a too high amount will result in side-effects of bloating and abdominal discomfort.

### Protein

The present composition comprises proteins. The protein component preferably provides 5 to 15% of the total calories. Preferably the present composition comprises a protein component that provides 6 to 12% of the total calories. More preferably protein is present in the composition below 9% based on calories, more preferably the composition comprises between 7.2 and 8.0% protein based on total calories, even more preferably between 7.3 and 7.7% based on total calories. A low protein concentration advantageously ensures a lower insulin response, thereby preventing proliferation of adipocytes in infants. Human milk comprises a lower amount of protein based on total calories than cow's milk. The protein concentration in a nutritional composition is determined by the sum of protein, peptides and free amino acids. Based on dry weight the composition preferably comprises less than 12 wt.% protein, more preferably between 9.6 to 12 wt.%, even more preferably 10 to 11 wt.%. Based on a ready-to-drink liquid product the composition preferably comprises less than 1.5 g protein per 100 ml, more preferably between 1.2 and 1.5 g, even more preferably between 1.25 and 1.35 g.

The source of the protein should be selected in such a way that the minimum requirements for essential amino acid content are met and satisfactory growth is ensured. Hence protein sources based on cows' milk proteins such as whey, casein and mixtures thereof and proteins based on soy, potato or pea are preferred. In case whey proteins are used, the protein source is preferably based on acid whey or sweet whey, whey protein isolate or mixtures thereof and may include α-lactalbumin and β-lactoglobulin. More preferably, the protein source is based on acid whey or sweet whey from which caseino-glyco-macropeptide (CGMP) has been removed. Preferably the composition comprises at least 3 wt.% casein based on dry weight. Preferably the casein is intact and/or non-hydrolyzed. For the present invention protein includes peptides and free amino acids.

### Other

The present composition is preferably particularly suitable for providing the daily nutritional requirements to a human with an age of at most 36 months of age, particularly at most 24 months of age, even more preferably at most 18 months of age, most preferably an infant with an age of below 12 months or below, most preferably from 0 to 6 months of age. Hence, the nutritional composition is for feeding or is used for feeding a human subject. The present invention thus also concerns a method of providing nutrition to a human subject with an age of 0 to 36 months, comprising oral administration of the nutritional composition according the present invention. In other words the invention concerns the use of lipid for the manufacture of a nutritional composition according to the present invention for providing nutrition to a human subject with an age of 0 to 36 months. In other words the invention concerns the nutritional composition according to the present invention for use in providing nutrition to a human subject with an age of 0 to 36 months.

The present composition comprises lipid, and protein and digestible carbohydrate wherein the lipid preferably provides 30 to 60 % of total calories, the protein preferably provides 5 to 20 %, more preferably 5 to 15 wt.%, of the total calories and the digestible carbohydrate preferably provides 25 to 75% of the total calories. Preferably the present composition comprises lipid providing 35 to 50% of the total calories, protein providing 6 to 12 % of the total calories and digestible carbohydrate providing 40 to 60 % of the total calories. In one embodiment, the protein provides 5 to 9 % of the total calories. The amount of total calories is determined by the sum of calories derived from protein, lipids and carbohydrates.

The composition of the invention preferably comprises other ingredients, such as vitamins, minerals according to international directives for infant formulae.

In one embodiment, the nutritional composition according to the invention or the nutritional composition for use according to the invention is a preterm formula, infant formula, follow on formula or growing up milk.

In order to meet the caloric requirements of the infant, the composition preferably comprises 45 to 200 kcal/100 ml liquid, more preferably 60 to 90 kcal/100 ml liquid, even more preferably 60 to 75 kcal/100 ml liquid. This caloric density ensures an optimal ratio between water and calorie consumption. The osmolarity of the present composition is preferably between 150 and 420 mOsmol/l, more preferably 260 to 320 mOsmol/1. The low osmolarity aims to reduce the gastrointestinal stress. Stress can induce adipocyte formation.

Preferably the composition is in a liquid form, with a viscosity below 35 mPa.s, more preferably below 6 mPa.s as measured in a Brookfield viscometer at 20°C at a shear rate of 100 s⁻¹. In one embodiment, the present composition is a powder. Suitably, the composition is in a powdered form, which can be reconstituted with water or other food grade aqueous liquid, to form a liquid, or in a liquid concentrate form that should be diluted with water. It was found that lipid globules maintained their size and coating when reconstituted. When the composition is in a liquid form, the preferred volume administered on a daily basis is in the range of about 80 to 2500 ml, more preferably about 450 to 1000 ml per day.

### Infant

Adipocytes proliferate during the first 36 months of life as well as more limited during puberty. The amount of adipocytes is an important determinant in the degree of fat mass, adipose tissue and/or obesity later-in-life. Hence the present composition is preferably administered to the human subject during the first 3 years of life. In one embodiment of the use according to the present invention, the nutritional composition is for feeding a human subject with an age between 0 and 36 months. It was found that there is a predominance of proliferation of adipocytes in the first 12 months of life with an optimum in perinatal adipocyte proliferation. Hence, it is particularly preferred that the present composition is administered to a human subject in this period of life. The present composition is therefore advantageously administered to a human of 0 to 24 months, more preferably to a human of 0 to 18 months, even more preferably to a human of 0 to 12 months, most preferably to a human of 0 to 6 months of age. The present invention particularly aims to prevent obesity later-in-life and is preferably not an obesity treatment. Hence, the present composition is preferably administered to an infant and/or toddler not suffering from obesity or overweight. In one embodiment of the use according to the present invention, the nutritional composition is for feeding a non-obese human subject. Preferably the composition is to be used in infants having a weight appropriate for gestational age.

Although the adipocyte proliferation is most pronounced during the first 36 months of life and puberty, adipocytes are formed also to a lesser degree in the interval between 36 months and puberty. So in one embodiment the present composition is preferably administered to an age up to 5 years, more preferably up to 10 years, more preferably up to 13 years.

### Obesity

Obesity in the present invention relates to an excess of body fat mass. Fat mass is also known as adipose tissue or fat tissue. An adult human person suffers from obesity if over 25 wt.% (for man) or over 30 wt.% (for women) of body weight is fat mass. Obesity is sometimes referred to as adiposity.

Suitable ways to determine % body fat mass are underwater weighing, skin fold measurement, bioelectrical impedance analysis, computed tomography (CT/CAT scan), magnetic resonance imaging (MRI/NMR), ultrasonography and dual energy X-ray absorptiometry (DEXA). A preferred method is DEXA measurement. In the context of this invention body fat mass is determined by DEXA.

The increased risk of health problems later in life, such as diabetes, in particular diabetes type 2, and metabolic syndrome, is related to the occurrence of visceral adiposity and not to general obesity. The term 'visceral obesity' refers to a condition with increased visceral fat tissue.

Visceral adiposity is typically caused by (accumulation of) excessive visceral fat tissue. Visceral fat, also known as organ fat, intra-abdominal fat, peritoneal fat or central fat, is normally located inside the peritoneal cavity as opposed to subcutaneous fat which is found underneath the skin and intramuscular fat which is found interspersed in skeletal muscles. Visceral fat includes the abdominal fat surrounding the vital organs and includes mesenteric fat, perirenal fat, retroperitoneal fat and preperitoneal fat (fat surrounding the liver). A waist circumference above 102 cm in adult man or above 88 cm in adult women indicates the presence of visceral adiposity. Hip-waist ratio's exceeding 0.9 in man and 0.85 in women indicate visceral adiposity. For children of 3-19 years old appropriate cutoffs for age- and sex-dependent waist circumferences can be found in Taylor et al, 2000 Am J Clin Nutr 72:490-495. A subject suffers from visceral adiposity when it meets one or more of the above criteria (regarding VAT, waist circumference or waist-hip ratio thresholds).

### Application

The present composition is preferably administered orally to the infant. The present invention also aims to prevent the occurrence of obesity and/or reduce the fat mass at the age above 36 months. In one embodiment the present method is for preventing obesity, reducing the risk of obesity and/or for improving body composition of a human subject when said human subject has an age above 36 months, preferably when said human subject has an age above 5 years, particularly above 13 years, more particularly above 18 years. In one embodiment the present method or the present nutritional composition is for feeding a human subject with an age between 0 and 36 months and for preventing obesity, reducing the risk of obesity and/or for improving body composition when said human subject has an age above 36 months, preferably to prevent obesity, reduce the risk of obesity and/or improve body composition at the age above 5 years, particularly above 13 years, more particularly above 18 years. In one embodiment the prevention of obesity, reduction of the risk of obesity and/or improving of body composition occurs later in life. With later in life is meant an age exceeding the age at which the diet is taken, preferably exceeding said age with at least one year. In one embodiment the present method or the present nutritional composition is for preventing visceral obesity and/or for reducing the ratio visceral fat to subcutaneous fat. The composition of the present invention therefore advantageously can be used for preventing obesity, reducing the risk of obesity later in life.

Likewise the composition can be used to prevent later in life diabetes mellitus type 2, decrease or prevent insulin resistance and/or improve insulin sensitivity, prevent or reduce the risk of metabolic syndrome and prevent or reduce the risk of osteopenia and/or osteoporosis, or reduce the risk of occurrence of osteopenia and/or osteoporosis. In one embodiment the present method or the present nutritional composition is for feeding a human subject with an age between 0 and 36 months and for prevention of diabetes type 2, reducing the risk of occurrence of insulin resistance and improving insulin sensitivity, preventing metabolic syndrome and preventing osteopenia and/or osteoporosis and/or for improving body composition when said human subject has an age above 36 months, preferably to prevent obesity, reduce the risk of obesity and/or improve body composition at the age above 5 years, particularly above 13 years, more particularly above 18 years. Not wishing to be bound by theory a reduced visceral obesity later in life causes these effects. Also improvement of pancreas development and liver function causes these effects.

The inventors surprisingly found that when mice during infancy and childhood were fed a food composition according to the present invention a different and significant effect on body composition later in life was observed compared to mice which during infancy and childhood had been fed a control food composition. Even after consumption of a Western style, mildly obesogenic diet. The composition of the present invention therefore advantageously can be used to improve body composition later in life, in particular increased lean body mass, decreased fat mass, decreased fat mass relative to total body weight, decreased visceral fat mass relative to total body weight, decreased visceral fat mass relative to total fat mass, decreased fat accumulation, increased muscle tissue. The composition of the present invention also advantageously can be used to increase bone mass and increase bone mineral density.

One embodiment of the use according to the present invention is for providing nutrition to a human subject with an age of 0 to 36 months.

Preterm and/or small for gestational age infants often encounter catch up growth early in life. This is generally seen as a risk factor for later in life adiposity. So the composition of the present invention is advantageously used in preterm infants or small for gestational age (SGA) infants, in particular for feeding a preterm infant or an infant small for gestational age. A preterm or premature infant relates to an infant born before the standard period of pregnancy is completed before or on 37 weeks pregnancy of the mother, i.e. before or on 37 weeks from the beginning of the last menstrual period of the mother. SGA babies are those whose birth weight lies below the 10th percentile for that gestational age. Premature and/or SGA infants include low birth weight infants (LBW infants), very low birth weight infants (VLBW infants), and extremely low birth weight infants (ELBW infants). LBW infants are defined as infants with a weight less than 2500 g. VLBW infants as infants with a weight which is less than 1500 g, and ELBW infants as infants with a weight less than 1000 g.

At day 70, which is a time point corresponding to late adolescence in humans, the mice, which had previously consumed the food composition of the present invention before turning to the Western style diet for 28 days, had a significantly lower fat mass accumulated and lower percentage fat mass based on body weight and increase muscle tissue than mice which had received a control composition during infancy. The total body weight and lean body mass was increased in the mice fed the experimental diet in early life. In particular visceral obesity was reduced. This is advantageous, since in particular visceral adipose tissue is most associated with health problems.

In this document and in its claims, the verb "to comprise" and its conjugations is used in its nonlimiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

### EXAMPLES

### Example 1: Effects of lipid globule size and source of triglyceride lipid on growth and body composition later in life

An experiment was performed wherein the effects of an infant milk formula (IMF) with standard vegetable lipid was compared with IMF wherein the lipid component a mixture of vegetable fat and milk fat.

C57/BL6 dams and their offspring were exposed to the diet from day 2 on. The offspring started eating the diet themselves from day 15 onward. They were completely fed on the diet from day 21 on. The experimental weaning diets were continued until day 42. From day 42 to day 70 all pups were fed the same diet based on AIN-93G diet with an adjusted lipid fraction (containing 20 wt.% lipid (17 wt % lard, 3 wt% soy oil, 0.1 wt% cholesterol). This diet represents a Western style diet.

The experimental diets comprised 282 g of experimental infant milk formula (IMF). The rest of the diet was AIN-93G protein, carbohydrates and fibre. All lipid present in the diet was derived from the IMF. The total amount of lipid was 7 wt% based on dry weight of the animal diet
The experimental IMF, which were present in the different diets, were prepared in a similar way as described in example 1B of WO 2010/0027259. Diet 2 was prepared with high pressure homogenization.

The detailed characteristics of the fat component of the different experimental IMFs used for the different animal diets are shown in Table 1. In the Western style diet the amount of LA was 11.9 wt.%, the amount of ALA was 1.3 wt.%, based on total fatty acids and the ratio LA/ALA was 9.15.

For diet 1 a mixture of palm oil, low erucic acid rape seed oil, coconut oil, high oleic sunflower oil, sunflower oil, with a small amount of soy lecithin and LC-PUFA premix was used, additionally comprised buttermilk powder as a source of milk derived phospholipids. The amount of vegetable fat in the final experimental IMF was about 94.6 wt% based on total fat. About 3.8 wt.% milk fat was present from the source of phospholipid, and traces form skim milk powder, and about 1.5 wt%. of a LC-PUFA premix comprising single cell oil and fish oil. The amount of milk derived phospholipids was about 1.49 wt.% based on total fat. The amount of soy phospholipids was about 0.13 wt.% based on total fat. The lipid globules had a volume mode diameter of about 3.6 µm and the volume % of lipid globules with a mode diameter between 2 and 12 µm was about 60.

For diet 2 a mix of anhydrous milk fat, coconut oil, low erucic acid rape seed oil, sunflower oil, high oileic acid sunflower oil, with a small amount of soy lecithin and LC-PUFA premix was used. The amount of vegetable fat was about 52.2 wt% based on total fat. The amount of anhydrous milk fat was about 46.3 %, the remainder about 1.5 wt% being fish oil and single cell oil as LC-PUFA source. The amount of soy phospholipids was about 0.13 wt.% based on total fat. The lipid globules had a volume mode diameter of about 0.46 µm and the volume % of lipid globules with a mode diameter between 2 and 12 µm was below about 10 %.

Diet 3 was similar to diet 2, but additionally comprised buttermilk powder as a source of milk derived phospholipids. The amount of vegetable fat was about 50.9 wt% based on total fat. Milk fat was present in about 47.6 wt% of which about 44.1 wt.% anhydrous milk fat. The remainder about 1.5 wt% being fish oil and single cell oil as LC-PUFA source. The amount of milk derived phospholipids was about 1.49 wt.% based on total fat. The amount of soy phospholipids was about 0.13 wt.% based on total fat.

The lipid globules had a volume mode diameter of about 5.6 µm and the volume % of lipid globules with a mode diameter between 2 and 12 µm was above 45.

**Table 1: Fatty acid profile of the lipid component of the diets, wt. % based on total fatty acid.**

| FA composition wt% | Diet 1 | Diet 2 | Diet 3 |
|---|---|---|---|
| C4:0 BA | 0.10 | 1.40 | 1.39 |
| C6:0 CA | 0.24 | 0.98 | 0.98 |
| C8:0 | 1.96 | 1.24 | 1.19 |
| C10:0 | 1.55 | 1.84 | 1.81 |
| C12:0 | 11.41 | 5.53 | 5.29 |
| C14:0 | 5.04 | 6.96 | 6.90 |
| C16:0 | 18.38 | 18.31 | 17.70 |
| C16:1 n7 | 0.23 | 0.94 | 0.95 |
| C18:0 | 3.58 | 6.07 | 6.23 |
| C18:1 n7 | 1.10 | 0.89 | 0.88 |
| C18:1 n9 | 37.27 | 34.07 | 34.73 |
| C 18:2n6 LA | 13.61 | 13.97 | 13.96 |
| C 18:3 n3 ALA | 2.53 | 2.59 | 2.59 |
| C20:0 | 0.34 | 0.31 | 0.31 |
| C20:1 n9 | 0.47 | 0.45 | 0.45 |
| C20:4n6 ARA | 0.36 | 0.35 | 0.36 |
| C20:5n3 EPA | 0.06 | 0.04 | 0.06 |
| C22:0 | 0.11 | 0.30 | 0.31 |
| C22:5 n3 DPA | 0.03 | 0.01 | 0.03 |
| C22:6 n3 DHA | 0.20 | 0.20 | 0.20 |
| Others | Up to 100 | Up to 100 | Up to 100 |
| SFA | 43.69 | 46.05 | 45.23 |
| MUFA | 39.32 | 36.70 | 37.38 |
| PUFA | 16.98 | 17.25 | 17.39 |
| LA/ALA | 5.4 | 5.4 | 5.4 |
| N6/n3 | 4.8 | 5.0 | 4.8 |
| LCPUFA | 0.69 | 0.68 | 0.71 |
| N6/n3 | 1.4 | 1.6 | 1.4 |
| SCFA | 0.33 | 2.38 | 2.37 |
| MCFA | 14.91 | 8.61 | 8.29 |

The mice were weighed twice a week. The food intake was determined once a week during the entire experiment. To determine body composition (i.e., BMC, BMD, fat mass (FM) and fat-free mass (FFM)) DEXA scans (Dual Energy X-ray Absorbiometry) were performed under general anesthesia at day 42, and 70 days after birth respectively, by densitometry using a PIXImus imager (GE Lunar, Madison, WI, USA).

The results are shown in Table 2.

**Table 2: Effects of early in life diets on development of body weight and composition.**

| | Day | 1 | 2 | 3 |
|---|---|---|---|---|
| BW g (s.e.) | 42 | 24.11 0.36 | 24.55 0.52 | 24.560.21 |
| | 70 | 29.81 (0.94) | 30.68 (0.90) | 30.24 (0.66) |
| LBM g (s.e.) | 42 | 19.47 0.42 | 20.07 0.56 | 20.020.34 |
| | 70 | 21.61 (0.51) | 22.73 (0.57) | 22.73 (0.39) |
| Fat g (s.e.) | 42 | 3.72 0.12 | 3.720.14 | 3.44 0.12 |
| | 70 | 24.83 (1.13) | 24.11 (1.25) | 23.59 (1.21) |
| Fat % of body weight (s.e.) | 42 | 15.99 0.32 | 15.62 0.40 | 14.70 0.57 |
| | 70 | 24.83 (1.13) | 24.11 (1.25) | 23.59 (1.21) |
| BMC g (s.e.) | 42 | 0.38 0.01 | 0.39 0.01 | 0.380.01 |
| | 70 | 0.44 (0.01) | 0.46 (0.01) | 0.470.01) |
| BMD g/cm² (s.e.) | 42 | 0.0465 0.0004 | 0.0469 0.0007 | 0.0473 0.0006 |
| | 70 | 0.052 (0.0008) | 0.053 (0.0008) | 0.055 (0.0008) |

As shown in Table 2 a combination of a vegetable fat and milk fat and a lipid globule increased in size and coated with phospholipids, or alternatively worded as a diet with about 2.4 wt% SCFA and about 17.4 wt% PUFA based on total fatty acids and a lipid globule increased in size and coated with phospholipids, while at the same time having a LA/ALA ratio of 5.4 (diet 3) showed at day 70 an improved effect on body composition later in life regarding increased lean body mass, decreased fat mass, decreased fat % of body mass, increased bone mineral content and increased bone mineral density, when compared with diet with a very small amount of milk fat due to the source of milk phospholipids, with SCFA below 0.4 wt% (diet 1) or with a diet with milk fat and vegetable fat, but with small (diameter 0.46 um) non coated lipid globules (diet 2). This effect was already present to a smaller extent on day 42, i.e. directly after having consumed the early in life diets. But strikingly the effects were even more pronounced at day 70, when the animals have been fed the same Western style diet for a while.

This is indicative for an improved effect on body composition and prevention of disorders, in particular later in life, such as obesity, metabolic syndrome, diabetes type 2 and osteoporosis, osteopenia when consuming a diet comprising a mixture of vegetable and milk lipid (or alternatively: of 0.6 to 4 wt.% short chain fatty acids based on total fatty acids) with a LA/ALA between 2 to 10, and with a lipid globule with a volume mode diameter above 1 µm, and with a fatty acid profile of the lipid comprises of 10 to 25 wt.% poly-unsaturated fatty acids (PUFA), based on total fatty acids.

The effect is further improved by the presence of 0.5 wt.% to 20 wt.% (based on total fat) of phospholipids coating the lipid globules

### Example 4: Infant formula.

An infant formula comprising per 100 ml 66 kcal, 1.3 g protein (cow's milk derived protein, with whey protein and casein in a weight ratio of 6:4), 7.3 g digestible carbohydrates (mainly lactose), 3.4 g lipid as described in diet 3 of example 2, 0.8 g non-digestible oligosaccharides (trans galacto-oligosaccharides and long chain fructo-oligosaccharide). Minerals, trace elements, vitamins, carnitine, choline, myo-inositol, and taurine as known in the art.

### Example 5: Infant formula

Infant formula according to example 4, except that now 36 wt.% anhydrous milk fat was used. The fatty acid profile based on total fatty acid comprises 17.0 wt.% PUFA, 14.3 wt.% LA, 2.6 wt.% ALA, 0.7 wt% LC-PUFA, 0.36 wt.% ARA, 0.2 wt.% DHA, 0.06 wt.% EPA and 0.03 wt% DPA, 1.1 wt.% BA, 0.8 wt% CA, and 14.1 wt% MCFA.

## Claims

1. Nutritional composition comprising carbohydrates, protein and lipid, wherein the lipid is present in the form of lipid globules, the lipid globules have a volume mode diameter of 1.0 µm or above, wherein the lipid has a fatty acid composition comprising:
i) 0.9 to 4 wt.% short chain fatty acids (SCFA) being the sum of butyric acid (BA) and caproic acid (CA) based on total fatty acyl chains, and wherein the composition comprises between 0.75 and 4 wt.% butyric acid based on weight of the total fatty acyl chains
ii) 10 to 25 wt.% poly-unsaturated fatty acids (PUFA), based on total fatty acids, comprising linoleic acid (LA) and alpha-linolenic acid (ALA) in a weight ratio of 2 to 10,
wherein the lipid is one or more selected from the group consisting of triglycerides, polar lipids, free fatty acids, mono- and diglycerides, and
wherein the lipid comprises 0.5 - 20 wt.% phospholipids based on total lipid.

2. Nutritional composition according to claim 1, wherein the lipid comprises 0.05 to 10 wt.% sphingomyelin based on total lipid.

3. Nutritional composition according to claim 1 or 2, wherein the phospholipid is present as a coating of the lipid globule.

4. Nutritional composition according to any one of the preceding claims, wherein the lipid comprises 0.25 wt. % to 5 wt.% LC-PUFA based on total fatty acids of which at least 0.15 wt% n-3 LC-PUFA based on total fatty acids selected from the group consisting of DHA, EPA, and DPA, more preferably DHA.

5. Nutritional composition according to any one of the preceding claims, wherein the lipid comprises of 5 to 15 wt.% medium chain fatty acids (MFCA) based on total fatty acids, wherein MCFA refer to fatty acids and/or acyl chains with a chain length of 8 to 12 carbon atoms.

6. Nutritional composition according to any one of the preceding claims, wherein the lipid comprises less than 15 wt.% linoleic acid and more than 1 wt.% alpha-linolenic acid based on total fatty acids.

7. Nutritional composition according to any one of the preceding claims, wherein the lipid globules have a volume mode diameter of above 2 µm.

8. Nutritional composition according to any one of the preceding claims wherein the composition comprises 10 to 50 wt.% lipids based on dry weight of the nutritional composition.

9. Nutritional composition according to any one of the preceding claims wherein the lipids provide 30 to 60 % of the total calories, the protein provides 5 to 20% of the total calories and the digestible carbohydrates provide 25 to 60% of the total calories.

10. Nutritional composition according to any one of the preceding claims, wherein the protein provides 5 to 9% of the total calories.

11. Nutritional composition according to any one of the preceding claims further comprising non digestible oligosaccharides, wherein the non-digestible oligosaccharides have a degree of polymerization (DP) between 2 and 250.

12. Nutritional composition according to any one of claims 1-11 for use in one or more selected from prevention of obesity, reducing the risk of obesity, prevention of diabetes type 2, reducing the risk of occurrence of insulin resistance and improving insulin sensitivity, preventing metabolic syndrome and preventing osteopenia and/or osteoporosis.

13. Non-therapeutic method for improving body composition, the improvement of body composition being selected from the group consisting of increased lean body mass, decreased fat mass relative to total body weight, decreased visceral fat mass relative to total body weight, a decreased visceral fat mass relative to total fat mass, decreased fat accumulation and increased muscle mass, increased bone mass and increased bone mineral density, comprising administering the nutritional composition according to any one of claims 1-11.

14. Nutritional composition for use according to claim 12, wherein the nutritional composition is for providing nutrition to a human subject with an age between 0 and 36 months.

15. Method according to claim 13, wherein the nutritional composition is for providing nutrition to a human subject with an age between 0 and 36 months.

16. Nutritional composition for use according to claim 14 wherein the prevention of obesity, reducing the risk of obesity, prevention of diabetes type 2, reducing the risk of occurrence of insulin resistance and improving insulin sensitivity, preventing metabolic syndrome and preventing osteopenia and/or osteoporosis occurs in a human subject when said human subject has an age above 36 months, preferably when said human subject has an age above 5 years.

17. Method according to claim 15, wherein improving body composition occurs in a human subject when said human subject has an age above 36 months, preferably when said human subject has an age above 5 years.

18. Nutritional composition for use according to any one of claims 12, 14 and 16, wherein the nutritional composition is selected from the group consisting of preterm formula, infant formula, follow on formula and growing up milk.

19. Method according to any one of claims 13, 15 and 17, wherein the nutritional composition is selected from the group consisting of preterm formula, infant formula, follow on formula and growing up milk.

20. Method for manufacture of nutritional composition according to claim 1 to 11 comprising the steps of preparing an aqueous phase comprising protein and digestible carbohydrates and preparing a fat phase comprising vegetable and milk lipids in a weight ratio of 3/7 to 9/1, wherein the fatty acid composition of the lipid comprises 10 to 25 wt.% poly-unsaturated fatty acids (PUFA), based on total fatty acids, comprising linoleic acid (LA) and alpha-linolenic acid (ALA) in a weight ratio of 2 to 10, comprising of 0.6 to 4 wt.% short chain fatty acids (SCFA) being the sum of butyric acid (BA) and caproic acid (CA)) based on total fatty acids, adding the fat and aqueous phase together and homogenizing the mixture of fat and aqueous phase into an oil-in-water emulsion with lipid globules having a volume mode diameter of 1.0 µm or above.

## Patentansprüche

1. Nahrungszusammensetzung, umfassend Kohlenhydrate, Protein und Lipid, wobei das Lipid in Form von Lipidkügelchen vorhanden ist, wobei die Lipidkügelchen einen Volumen-Modus-Durchmesser von 1,0 µm oder größer aufweisen, wobei das Lipid eine Fettsäurezusammensetzung aufweist, umfassend:
i) 0,9 bis 4 Gew.-% kurzkettige Fettsäuren (SCFA), wobei es sich um die Summe von Buttersäure (BA) und Capronsäure (CA) handelt, bezogen auf die gesamten Fettacylketten, und wobei die Zusammensetzung zwischen 0,75 und 4 Gew.-% Buttersäure, bezogen auf das Gewicht der gesamten Fettacylketten, umfasst
ii) 10 bis 25 Gew.-% mehrfach ungesättigte Fettsäuren (PUFA), bezogen auf die Gesamtfettsäuren, umfassend Linolsäure (LA) und Alpha-Linolensäure (ALA) in einem Gewichtsverhältnis von 2 bis 10,
wobei es sich bei dem Lipid um eines oder mehrere, ausgewählt aus der Gruppe bestehend aus Triglyceriden, polaren Lipiden, freien Fettsäuren, Mono- und Diglyceriden, handelt, und
wobei das Lipid 0,5 - 20 Gew.-% Phospholipide, bezogen auf das Gesamtlipid, umfasst.

2. Nahrungszusammensetzung gemäß Anspruch 1, wobei das Lipid 0,05 bis 10 Gew.-% Sphingomyelin, bezogen auf das Gesamtlipid, umfasst.

3. Nahrungszusammensetzung gemäß Anspruch 1 oder 2, wobei das Phospholipid als ein Überzug des Lipidkügelchens vorhanden ist.

4. Nahrungszusammensetzung gemäß einem der vorangehenden Ansprüche, wobei das Lipid 0,25 Gew.-% bis 5 Gew.-% LC-PUFA, bezogen auf die Gesamtfettsäuren, umfasst, von welchem wenigstens 0,15 Gew.-% n-3 LC-PUFA, bezogen auf die Gesamtfettsäuren, ausgewählt aus der Gruppe bestehend aus DHA, EPA und DPA, mehr bevorzugt DHA ist.

5. Nahrungszusammensetzung gemäß einem der vorangehenden Ansprüche, wobei das Lipid 5 bis 15 Gew.-% mittelkettige Fettsäuren (MFCA), bezogen auf die Gesamtfettsäuren, umfasst, wobei sich MCFA auf Fettsäuren und/oder Acylketten mit einer Kettenlänge von 8 bis 12 Kohlenstoffatomen bezieht.

6. Nahrungszusammensetzung gemäß einem der vorangehenden Ansprüche, wobei das Lipid weniger als 15 Gew.-% Linolsäure und mehr als 1 Gew.-% Alpha-Linolensäure, bezogen auf die Gesamtfettsäuren, umfasst.

7. Nahrungszusammensetzung gemäß einem der vorangehenden Ansprüche, wobei die Lipidkügelchen einen Volumen-Modus-Durchmesser von mehr als 2 µm aufweisen.

8. Nahrungszusammensetzung gemäß einem der vorangehenden Ansprüche, wobei die Zusammensetzung 10 bis 50 Gew.-% Lipide, bezogen auf das Trockengewicht der Nahrungszusammensetzung, umfasst.

9. Nahrungszusammensetzung gemäß einem der vorangehenden Ansprüche, wobei die Lipide 30 bis 60 % der Gesamtkalorien bereitstellen, das Protein 5 bis 20 % der Gesamtkalorien bereitstellt und die verdaulichen Kohlenhydrate 25 bis 60 % der Gesamtkalorien bereitstellen.

10. Nahrungszusammensetzung gemäß einem der vorangehenden Ansprüche, wobei das Protein 5 bis 9 % der Gesamtkalorien bereitstellt.

11. Nahrungszusammensetzung gemäß einem der vorangehenden Ansprüche, außerdem umfassend unverdauliche Oligosaccharide, wobei die unverdaulichen Oligosaccharide einen Polymerisationsgrad (DP) zwischen 2 und 250 aufweisen.

12. Nahrungszusammensetzung gemäß einem der Ansprüche 1-11 zur Verwendung bei einem oder mehreren, ausgewählt aus Verhütung von Fettleibigkeit, Verringerung des Risikos von Fettleibigkeit, Verhütung von Diabetes Typ 2, Verringerung des Risikos des Auftretens einer Insulinresistenz und Verbesserung der Insulinempfindlichkeit, Verhütung des metabolischen Syndroms und Verhütung von Osteopenie und/oder Osteoporose.

13. Nicht-therapeutisches Verfahren zum Verbessern der Körperzusammensetzung, wobei die Verbesserung der Körperzusammensetzung ausgewählt ist aus der Gruppe bestehend aus einer erhöhten mageren Körpermasse, herabgesetzten Fettmasse, bezogen auf das Gesamtkörpergewicht, herabgesetzten Masse des viszeralen Fetts, bezogen auf das Gesamtkörpergewicht, einer herabgesetzten Masse des viszeralen Fetts, bezogen auf die Gesamtfettmasse, einer herabgesetzten Fettansammlung und erhöhten Muskelmasse, erhöhten Knochenmasse und erhöhten Knochenmineraldichte, umfassend das Verabreichen der Nahrungszusammensetzung gemäß einem der Ansprüche 1-11.

14. Nahrungszusammensetzung zur Verwendung gemäß Anspruch 12, wobei die Nahrungszusammensetzung zum Bereitstellen von Nahrung für einen menschlichen Probanden mit einem Alter zwischen 0 und 36 Monaten bestimmt ist.

15. Verfahren gemäß Anspruch 13, wobei die Nahrungszusammensetzung zum Bereitstellen von Nahrung für einen menschlichen Probanden mit einem Alter zwischen 0 bis 36 Monaten bestimmt ist.

16. Nahrungszusammensetzung zur Verwendung gemäß Anspruch 14, wobei die Verhütung von Fettleibigkeit, Verringerung des Risikos von Fettleibigkeit, Verhütung von Diabetes Typ 2, Verringerung des Risikos des Auftretens einer Insulinresistenz und Verbesserung der Insulinempfindlichkeit, Verhütung des metabolischen Syndroms und Verhütung von Osteopenie und/oder Osteoporose bei einem menschlichen Probanden stattfindet, wenn der menschliche Proband ein Alter über 36 Monate aufweist, vorzugsweise wenn der menschliche Proband ein Alter über 5 Jahre aufweist.

17. Verfahren gemäß Anspruch 15, wobei das Verbessern der Körperzusammensetzung bei einem menschlichen Probanden stattfindet, wenn der menschliche Proband ein Alter über 36 Monate aufweist, vorzugsweise wenn der menschliche Proband ein Alter über 5 Jahre aufweist.

18. Nahrungszusammensetzung zur Verwendung gemäß einem der Ansprüche 12, 14 und 16, wobei die Nahrungszusammensetzung aus der Gruppe bestehend aus Frühgeborenennahrung, Säuglingsnahrung, Folgenahrung und Kleinkindmilch ausgewählt ist.

19. Verfahren gemäß einem der Ansprüche 13, 15 und 17, wobei die Nahrungszusammensetzung aus der Gruppe bestehend aus Frühgeborenennahrung, Säuglingsnahrung, Folgenahrung und Kleinkindmilch ausgewählt ist.

20. Verfahren zum Herstellen einer Nahrungszusammensetzung gemäß Anspruch 1 bis 11, umfassend die Schritte des Herstellens einer wässrigen Phase, die Protein und verdauliche Kohlenhydrate umfasst, und des Herstellens einer Fettphase, die pflanzliche und Milchlipide in einem Gewichtsverhältnis von 3/7 bis 9/1 umfasst, wobei die Fettsäurezusammensetzung des Lipids 10 bis 25 Gew.-% mehrfach ungesättigte Fettsäuren (PUFA), bezogen auf die Gesamtfettsäuren, umfasst, umfassend Linolsäure (LA) und Alpha-Linolensäure (ALA) in einem Gewichtsverhältnis von 2 bis 10, umfassend 0,6 bis 4 Gew.-% kurzkettige Fettsäuren (SCFA), wobei es sich um die Summe von Buttersäure (BA) und Capronsäure (CA) handelt, bezogen auf die Gesamtfettsäuren, Zusammengeben der Fettphase und der wässrigen Phase und Homogenisieren der Mischung aus Fettphase und wässriger Phase zu einer Ölin-Wasser-Emulsion mit Lipidkügelchen, die einen Volumen-Modus-Durchmesser von 1,0 µm oder größer aufweisen.

## Revendications

1. Composition nutritionnelle comprenant des glucides, des protéines et des lipides, dans laquelle le lipide est présent sous la forme de globules lipidiques, les globules lipidiques ayant un diamètre en mode volume de 1,0 µm ou plus, dans laquelle le lipide a une composition d'acides gras comprenant :
i) 0,9 à 4 % en poids d'acides gras à chaîne courte (SCFA) étant la somme de l'acide butyrique (BA) et de l'acide caproïque (CA) sur la base des chaînes d'acyles gras totales, et dans laquelle la composition comprend entre 0,75 et 4 % en poids d'acide butyrique sur la base du poids des chaînes d'acyle gras totales,
ii) 10 à 25 % en poids d'acides gras poly-insaturés (PUFA), sur la base des acides gras totaux, comprenant de l'acide linoléique (LA) et de l'acide alpha-linolénique (ALA) selon un rapport pondéral de 2 à 10,
dans laquelle le lipide est un ou plusieurs choisis dans le groupe constitué de triglycérides, de lipides polaires, d'acides gras libres, de mono- et diglycérides, et
dans lequel le lipide comprend 0,5 à 20 % en poids de phospholipides par rapport au lipide total.

2. Composition nutritionnelle selon la revendication 1, dans laquelle le lipide comprend 0,05 à 10 % en poids de sphingomyéline sur la base du lipide total.

3. Composition nutritionnelle selon la revendication 1 ou 2, dans laquelle le phospholipide est présent en tant que revêtement du globule lipidique.

4. Composition nutritionnelle selon l'une quelconque des revendications précédentes, dans laquelle le lipide comprend de 0,25 % à 5 % en poids de LC-PUFA sur la base des acides gras totaux, dont au moins 0,15 % en poids de LC-PUFA n-3 sur la base des acides gras totaux choisis dans le groupe constitué de DHA, EPA et DPA, de manière plus préférée DHA.

5. Composition nutritionnelle selon l'une quelconque des revendications précédentes, dans laquelle le lipide comprend de 5 à 15 % en poids d'acides gras à chaîne moyenne (MFCA) sur la base des acides gras totaux, dans laquelle MCFA désigne des acides gras et/ou des chaînes acyles ayant une longueur de chaîne de 8 à 12 atomes de carbone.

6. Composition nutritionnelle selon l'une quelconque des revendications précédentes, dans laquelle le lipide comprend moins de 15 % en poids d'acide linoléique et plus de 1 % en poids d'acide alpha-linolénique sur la base des acides gras totaux.

7. Composition nutritionnelle selon l'une quelconque des revendications précédentes, dans laquelle les globules lipidiques ont un diamètre en mode volume supérieur à 2 µm.

8. Composition nutritionnelle selon l'une quelconque des revendications précédentes, dans laquelle la composition comprend 10 à 50 % en poids de lipides sur la base du poids sec de la composition nutritionnelle.

9. Composition nutritionnelle selon l'une quelconque des revendications précédentes, dans laquelle les lipides fournissent 30 à 60 % des calories totales, la protéine fournit 5 à 20 % des calories totales et les glucides digestibles fournissent 25 à 60 % des calories totales.

10. Composition nutritionnelle selon l'une quelconque des revendications précédentes, dans laquelle la protéine fournit 5 à 9 % des calories totales.

11. Composition nutritionnelle selon l'une quelconque des revendications précédentes, comprenant en outre des oligosaccharides non digestibles, dans laquelle les oligosaccharides non digestibles ont un degré de polymérisation (DP) compris entre 2 et 250.

12. Composition nutritionnelle selon l'une quelconque des revendications 1 à 11, à utiliser dans une ou plusieurs parmi la prévention de l'obésité, la réduction du risque d'obésité, la prévention du diabète de type 2, la réduction du risque d'apparition d'une résistance à l'insuline et l'amélioration de la sensibilité à l'insuline, la prévention du syndrome métabolique et la prévention de l'ostéopénie et/ou de l'ostéoporose.

13. Méthode non thérapeutique pour améliorer la composition corporelle, l'amélioration de la composition corporelle étant choisie dans le groupe comprenant une masse corporelle accrue, une masse grasse diminuée par rapport au poids de corps total, une masse grasse viscérale réduite par rapport au poids de corps total, une masse grasse viscérale réduite par rapport à la masse grasse totale, une accumulation de graisse réduite et une masse musculaire accrue, une masse osseuse accrue et une densité minérale osseuse accrue, comprenant l'administration de la composition nutritionnelle selon l'une quelconque des revendications 1 à 11.

14. Composition nutritionnelle à utiliser selon la revendication 12, dans laquelle la composition nutritionnelle est destinée à fournir une nutrition à un sujet humain âgé de 0 à 36 mois.

15. Méthode selon la revendication 13, dans laquelle la composition nutritionnelle est destinée à fournir une nutrition à un sujet humain âgé de 0 à 36 mois.

16. Composition nutritionnelle à utiliser selon la revendication 14, dans laquelle la prévention de l'obésité, la réduction du risque d'obésité, la prévention du diabète de type 2, la réduction du risque de résistance à l'insuline et l'amélioration de la sensibilité à l'insuline, la prévention du syndrome métabolique et la prévention de l'ostéopénie et/ou l'ostéoporose survient chez un sujet humain lorsque ledit sujet humain a un âge supérieur à 36 mois, de préférence lorsque ledit sujet humain a un âge supérieur à 5 ans.

17. Méthode selon la revendication 15, dans laquelle l'amélioration de la composition corporelle survient chez un sujet humain lorsque ledit sujet humain a un âge supérieur à 36 mois, de préférence lorsque ledit sujet humain a un âge supérieur à 5 ans.

18. Composition nutritionnelle à utiliser selon l'une quelconque des revendications 12, 14 et 16, dans laquelle la composition nutritionnelle est choisie dans le groupe constitué par des préparations pour prématurés, des préparations pour nourrissons, des préparations de suite et du lait de croissance.

19. Procédé selon l'une quelconque des revendications 13, 15 et 17, dans lequel la composition nutritionnelle est choisie dans le groupe constitué par les préparations pour prématurés, les préparations pour nourrissons, les préparations pour suite et le lait de croissance.

20. Procédé de fabrication d'une composition nutritionnelle selon les revendications 1 à 11, comprenant les étapes consistant à préparer une phase aqueuse comprenant des protéines et des glucides digestibles et à préparer une phase grasse comprenant des lipides végétaux et de lait selon un rapport pondéral de 3/7 à 9/1, dans laquelle la composition d'acides gras du lipide comprend 10 à 25 % en poids d'acides gras poly-insaturés (PUFA), sur la base des acides gras totaux, comprenant de l'acide linoléique (LA) et de l'acide alpha-linolénique (ALA) selon un rapport pondéral de 2 à 10, comprenant de 0,6 à 4 % en poids d'acides gras à chaîne courte (SCFA) étant la somme de l'acide butyrique (BA) et de l'acide caproïque (CA) sur la base des acides gras totaux, à ajouter la matière grasse et la phase aqueuse ensemble et à homogénéiser le mélange de matière grasse et de phase aqueuse dans une émulsion huile dans eau avec des globules lipidiques ayant un diamètre en mode volume de 1,0 ou plus.
